(19) Europäisches Patentamt
European Patent Office
Office européen des brevets

(11) Numéro de publication : **0 310 645 B1**

# (12) FASCICULE DE BREVET EUROPEEN

(45) Date de publication du fascicule du brevet : **10.06.92 Bulletin 92/24**

(51) Int. Cl.$^5$ : **C07B 59/00,** A61K 43/00, A61K 49/02

(21) Numéro de dépôt : **88903292.6**

(22) Date de dépôt : **15.04.88**

(86) Numéro de dépôt international : **PCT/FR88/00188**

(87) Numéro de publication internationale : **WO 88/07986 20.10.88 Gazette 88/23**

## (54) COMPOSES UTILES NOTAMMENT POUR LA RADIOTHERAPIE OU L'IMAGERIE DU CANCER.

Le dossier contient des informations techniques présentées postérieurement au dépôt de la demande et ne figurant pas dans leprésent fascicule.

(30) Priorité : **17.04.87 FR 8705524**

(43) Date de publication de la demande : **12.04.89 Bulletin 89/15**

(45) Mention de la délivrance du brevet : **10.06.92 Bulletin 92/24**

(84) Etats contractants désignés : **AT BE CH DE FR GB IT LI LU NL SE**

(56) Documents cités :
**EP-A- 0 169 515**
**EP-A- 0 190 905**
**WO-A-70/6138**
**DE-A- 2 518 547**

(56) Documents cités :
**International Journal of Radiation Applications and Instrumentation. Part A, Applied Radiation and Isotopes, vol. 37, no. 8,1986, Marsh Barton, Essex (GB) H.K. Misra et al.: "Synthesis of 131I, 123I, and 82BR labelled5-halo-1-(2-deoxy-2fluoro-B-D-arabinofuranosyl) uracils", pages 901-905, see the whole article**
**Journal of Nuclear Medicine, vol. 25, no. 10, October 1984, (New York, US) L.A. Franke et al.: "Radioiodinated ligands forthe estrogen receptor: effect of 3-methylation of tissue distribution", pages 1116-1121**
**Steroids, vol. 38, no. 5, November 1981, (San Francisco, US) T. Ratajczak et al.: "The synthesis and study of some potentialaffinity labelling reagents for estrogen receptors", pages 537-555**

(73) Titulaire : **IRE-CELLTARG S.A.**
**B-6220 Fleurus (BE)**

(72) Inventeur : **QUIVY, Jacques**
**22, cours du Cramignon**
**B-1348 Ottignies/Louvain-la-Neuve (BE)**
Inventeur : **ZEICHER, Marc**
**18, rue Auguste Danse**
**B-1180 Bruxelles (BE)**
Inventeur : **WORCEL, Manuel**
**36, rue de Pommard**
**F-75012 Paris (FR)**

(74) Mandataire : **Warcoin, Jacques et al**
**Cabinet Régimbeau 26, avenue Kléber**
**F-75116 Paris (FR)**

**EP 0 310 645 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

Le domaine technique de la présente invention est celui de la thérapie ciblée et de l'imagerie médicale, notamment du cancer.

En particulier, la présente invention concerne le traitement des cancers disséminés à l'aide de radioisotopes cytotoxiques ciblés.

Selon l'invention, la cible pour les radiations ionisantes administrées est l'A.D.N. des cellules malignes. L'effet stérilisant des radiations est dû de façon prépondérante à l'induction de doubles cassures dans l'A.D.N..

L'utilisation de radioisotopes se désintégrant en émettant des électrons Auger est très propice à cet effet. Due au court parcours des électrons AUGER, l'efficacité de tels radioisotopes, en ce qui concerne l'inactivité cellulaire, est totalement perdue lorsqu'ils ne sont pas liés ou tout au plus à une distance de quelques atomes de l'A.D.N..

Par contre, de tels radioisotopes deviennent très efficaces lorsqu'ils sont liés à des molécules s'incorporant dans l'A.D.N. ou se fixant à l'A.D.N..

$^{125}I$ et $^{80m}Br$ ont déjà été proposés dans ce but. Cependant, $^{125}I$ n'est pas idéal pour cette application in-vivo vu sa demi-vie de 60 jours.

Par exemple, le turn-over des complexes oestrogènes récepteurs d'oestrogènes prend place à une échelle d'heures et non de jours, ce qui signifie que seulement une faible fraction de la radioactivité administrée au patient atteindra les cellules cibles au moment opportun et que le patient sera exposé inutilement à une quantité de radioactivité importante.

Le brome $80^m$ par contre a une demi-vie de 4,4 heures, ce qui impose pratiquement de traiter le patient dans les environs immédiats du cyclotron. En outre, le $Br80^m$ ne produit qu'une moyenne de 6 à 7 électrons AUGER par désintégration.

On a découvert, selon l'invention, que l'iode 123 qui a une demi-vie de 13, 21 heures et produit environ une vingtaine d'électrons ALGER par désintégration, ne présente pas les inconvénients de l'iode 125 et du Brome $80^m$. Sa demi-vie est suffisamment courte pour ne pas exposer inutilement le patient à des doses de radioactivité pendant une période prolongée mais est suffisamment longue pour permettre la synthèse des produits radiopharmaceutiques et leur envoi dans les centres de traitement situés loin du cylcotron de production, compte tenu du fait qu'il faut prévoir jusqu'à 48 heures de trajet et 24 heures de traitement.

En effet, les activités spécifiques maximales pour un atome d'halogène radioactif incorporé par molécule sont les suivantes :

| | | | |
|---|---|---|---|
| pour le Br $80^m$ | 0 h | 712.415 | Ci/mmole |
| | 24 h | 16.259 | Ci/mmole |
| $N_{24h} = N_o \times \frac{1}{43,816}$ | 48 h | 371 | Ci/mmole |
| | 72 h | 8 | Ci/mmole |
| pour $I^{123}$ | 0 h | 237.292 | Ci/mmole |
| | 24 h | 67.373 | Ci/mmole |
| $N_{24h} = N_o \times \frac{1}{3,522}$ | 48 h | 19.129 | Ci/mmole |
| | 72 h | 5.431 | Ci/mmole |
| pour $I^{125}$ | 0 h | 2.177 | Ci/mmole |
| $N_{24h} = N_o \times \frac{1}{1,0116}$ | 24 h | 2.152 | Ci/mmole |
| | 48 h | 2.127 | Ci/mmole |
| | 72 h | 2.103 | Ci/mmole |
| | | | Ci (curie) |

En outre, l'activité décroît très vite après 72 heures pour $I^{123}$ alors qu'elle persiste avec $I^{125}$ laissant subs-

tituer une contamination inutile.

C'est pourquoi, la présente invention a pour objet un composé utile notamment pour le traitement en radiothérapie ciblée ou l'imagerie du cancer, caractérisé en ce qu'il est constitué par une molécule susceptible de passer à proximité de l'ADN des cellules cibles, ladite molécule étant radiomarquée à l'iode 123.

On peut également citer comme molécules susceptibles de passer à proximité de l'ADN des cellules cibles des ligands spécifiques de récepteurs d'hormones stéroïdes.

Les hormones stéroïdes se lient avec une haute affinité aux récepteurs protéiques cytoplasmiques des cellules cibles et, après liaison, subissent une translocation dans le noyau cellulaire et activent la transcription des portions du génome relatives à l'effet physiologique propre à l'hormone. Etant donné le passage du complexe stéroïde-récepteur à proximité du DNA, un iode radioactif $I^{125}$ ou $I^{123}$ porté par le stéroïde pourra endommager gravement le DNA et avoir un effet léthal sur la cellule cible.

Or un certain nombre de cancers présentent une concentration élevée en récepteurs spécifiques soit aux estrogènes, soit aux progestagènes, soit aux androgènes. Il s'agit notamment des cancers du sein, de l'utérus, de l'ovaire et de la prostate. Par example, 65 % des cancers du sein présentent des niveaux détectables de récepteurs d'estrogènes (de 5000 à 50000 molécules de récepteurs par cellule).

Un iode radioactif $I^{125}$ ou $I^{123}$ attaché au stéroïde permettra la destruction spécifique des cellules cancéreuses. En outre, un iode 123 permettra de visualiser la tumeur par radioimagerie. En fait, ces ligands constituent des agents de pilotage de l'élément actif proprement dit qui est leur radionucléide.

Pour des sites de fixation intracellulaire tels que les récepteurs hormonaux, des analogues d'hormones présentant une haute affinité pour le récepteur et une faible affinité pour les proteines de liaison plasmatiques seront les agents de pilotage les plus appropriés.

Un composé 11-beta-chlorométhyl-estradiol est décrit dans "Steroids", 38, n° 5, 1981, pages 537-555. EP-A-169515 décrit des composés 17-alpha-iodovinyl-oestradiol.

La présente invention concerne de nouveaux ligands stéroïdes spécifiques de récepteurs hormonaux, ligands pouvant alors porter un radionucléide $I^{125}$ ou $I^{123}$.

Un but de la présente invention était aussi, en effet, de proposer de nouveaux analogues d'hormo nes stéroïdes présentant une haute affinité pour le récepteur hormonal et qui se lie de manière quasi irréversible au récepteur en vue d'améliorer la proportion d'analogues fixés, les dérivés iodés de ces analogues devant être stables dans le milieu extracellulaire, notamment dans le plasma, comme dans le cytoplasme, tout en gardant une haute affinité pour le récepteur hormonal.

Toutes les cellules contenant des récepteurs hormonaux, qu'elles soient malignes ou saines, peuvent être l'objet de cet accroissement de cytotoxicité.

Toutefois, on observe quand même une sélectivité améliorée, puisque dans les traitements de chimiothérapie de cancer conventionnel, les tissus cibles estrogènes sains ont soit un taux de prolifération de cellules faibles, soit ne sont pas essentiels à la vie. Il en est de même pour les récepteurs androgènes et progestagènes.

La présente invention a donc également pour objet de nouveaux ligands spécifiques de récepteurs d'hormones stéroides utiles pour la thérapie ciblée ou l'imagerie notamment du cancer présentant un squelette structurel de base ayant pour formule :

(I)

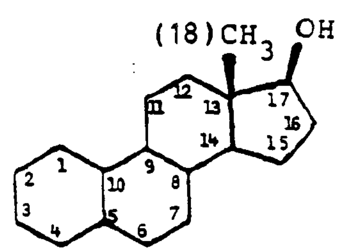

Ces produits de formule I sont caractérisés en ce qu'ils comportent notamment :

a une fonction hydroxyle ou cétone en position $C_3$,

b une fonction chlorométhyle en β sur la position $C_{11}$,

c une fonction vinyle ou méthyle en α sur la position $C_{17}$ et,

d un iode radioactif substitué sur un groupement alkyl ou alkényl rattaché au squelette, notamment un méthyl ou un vinyl.

Les fonctions en positions $C_3$ et $C_{17}$ confèrent à ces ligands une affinité élevée pour les récepteurs hormonaux auxquels ils sont destinés, à savoir des récepteurs androgènes, estrogènes ou progestagènes notamment.

La fonction 11 β-chlorométhyle assure une liaison quasi "irréversible" entre le stéroïde et le récepteur, il

s'agit en fait plutôt d'une très forte affinité pour le récepteur.

La formation du lien quasi "irréversible" entre le stéroïde et le récepteur améliore la stabilité du complexe et augmente sa concentration dans le noyau.

Les sites de liaison des hormones estrogènes, androgènes et progestagènes présentent des homologies de séquences d'acide aminé, ce qui explique leur commun comportement s'agissant de la liaison"irréversible"entre le récepteur et le stéroïde en présence d'une fonction 11 β-chlorométhyle.

Ces dérivés possèdent une fonction vinyle ou méthyle en $C_{17}$ en α de la position $C_{17}$, ce qui leur confère une résistance au métabolisme et une liaison réduite au protéine de liaison plasmatique.

Les ligands, plus particulièrement utiles pour la thérapie ciblée, comporteront l'isotope $I^{125}$ ou $I^{123}$ comme iode radioactif.

Cet iode radioactif ainsi piloté présente une forte activité cytotoxique, dans la mesure où il est amené à proximité de l'ADN dont il induit des coupures dans la double hélice.

Des ligands, plus particulièrement utiles pour l'imagerie médicale, notamment du cancer, porteront comme iode radioactif l'isotope $I^{123}$ .

Selon une première variante des ligands selon l'invention, l'iode radioactif est situé sur la double liaison du vinyl en α sur la position $C_{17}$. Ces ligands sont plus spécifiques des récepteurs estrogènes et progestagènes. Cette position a l'avantage de présenter une grande stabilité.

Selon une seconde variante des ligands selon l'invention, l'iode radioactif est situé sur un méthyl angulaire positionné en $C_{18}$. La position $C_{18}$ est celle qui est directement liée à $C_{13}$. Ces ligands sont plus particulièrement spécifiques des récepteurs androgènes.

On peut citer en particulier comme ligands selon la première variante, ceux dont la fonction en $C_3$ est un groupe hydroxyl, le cycle sur lequel il est attaché étant un cycle aromatique. Ces ligands sont plus spécifiques des récepteurs estrogènes.

On peut également citer comme ligands selon la première variante de l'invention des ligands dont la fonction en position $C_3$ est une fonction cétone conjuguée à une double liaison en $C_4$-$C_5$. Ces ligands sont plus particulièrement spécifiques des récepteurs progestagènes.

Parmi les ligands, plus particulièrement spécifiques des récepteurs androgènes, dont l'iode radioactif est situé sur un méthyl angulaire positionné en $C_{18}$, on peut citer ceux dont la fonction en position $C_3$ est une cétone conjuguée à une double liaison en $C_4$-$C_5$.

Parmi ces derniers, on peut également citer ceux qui comportent un méthyle en position $C_7$.

Parmi les ligands, plus spécifiques des récepteurs estrogènes vus précédemment, on peut citer le 11 β-chlorométhyl 17 α-iodovinyl estradiol de formule

(II)

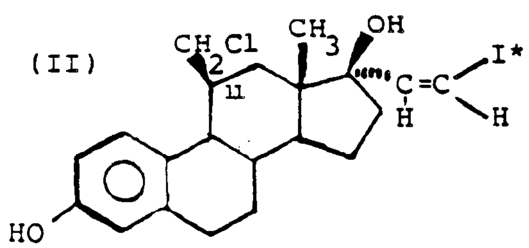

Parmi les ligands les plus spécifiques des récepteurs progestaqènes, on peut citer particulièrement le 11 β-chlorométhyl 17 α-iodovinyl 17 β-hydroxy 19-nor 4-androstène 3-one de formule

(III)

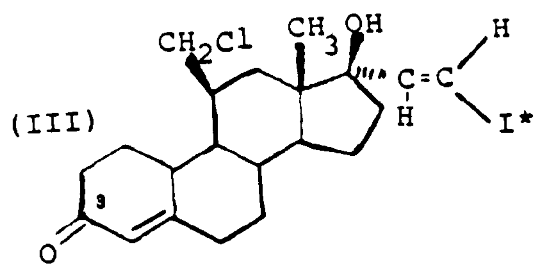

Parmi les ligands spécifiques des récepteurs androgènes, on peut citer les 11 β-chlorométhyle 17 α-méthyle 18-iodotestostérone ou 19-nortestostérone de formule

(IV)

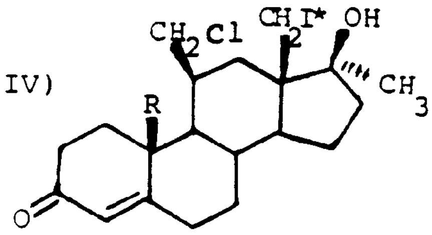

selon que
R = $CH_3$ (dérivé testostérone) ou
H (dérivé 19-nortestostérone)

Parmi ces derniers, on peut également citer les 11 β-chlorométhyle 17 α-méthyle 18-iodo 7 α-méthyle testostérone ou 19-nortestostérone de formule

(V)

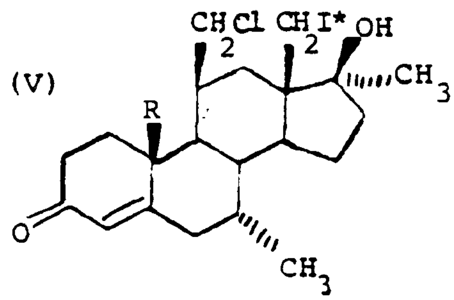

selon que
R = $CH_3$ (dérivé testostérone) ou
H (dérivé 19-nortestostérone)

D'autres caractéristiques et avantages de la présente invention apparaîtront à la lumière de la description qui va suivre.

Exemple III: LIGANDS SPECIFIQUES DU RECEPTEUR ESTROGENE

Le schéma I ci-après reproduit la synthèse d'un ligand 11 β-chlorométhyl 17 α-iodovinyl-estradiol.

L'étape 1 consiste en une hydroxylation microbiologique avec une souche "ASPERGILLUS OCHRACEUS" en 11 α selon les détails fournis page 136 de la référence 4. Le produit de départ est le 17 α-éthynyl estradiol [1].

L'étape 2 est une oxydation avec du pyridinium chlorochromate (PCC) dans $CH_2Cl_2$ oxydation qui donne donc une fonction cétone en position 11 selon le protocole décrit page 2649 de la référence 7.

L'étape 3 consiste en une protection avec le 1 éthoxy-éthyl-éther par action de l'éthyl-vinyl-éther dans la fonction hydroxyl en position $C_3$. Le mode opératoire à suivre est décrit page 334 de la référence 2.

L'étape 4 consiste en une méthylénation de la fonction cétone en position $C_{11}$ par addition de$(CH_3)_3$ Si $CH_3$ Mg Cl dont les détails sont décrits page 39 de la référence 3.

L'étape 5 consiste en une déshydratation avec un mélange HCl acétone conformément au mode opératoire détaillé page 39 de la référence 3 et une déprotection des fonctions en $C_3$ et $C_{17}$.

L'étape 6 consiste en une hydroboration sélective de la double liaison en $C_{11}$ avec le 9-borabicyclo (3.3.1) nonane (BBN) dont les détails sont fournis en référence 8 .

L'étape 7 consiste en une hydroxylation du dérivé BBN par oxydation avec de la soude (NaOH) et de l'eau oxygénée ($H_2O_2$). Ce type d'hydroxylation est décrit page 334 de la référence 2.

L'étape 8 est une chloration par du triphényl phosphine et du N-chlorosuccinimide (NCS) conformément au procédé décrit page 500 de la référence 5. On obtient donc un dérivé 11 β-chlorométhyl.

L'étape 9 est une hydroboration par catéchol borane à 70°C pendant 2 heures selon le procédé décrit page 1289 de la référence 6. On obtient donc un dérivé 17 α-vinyl.

L'étape 10 est une formation d'un dérivé acétate en position $C_3$ avec de l'anhydride acétique et de la pyridine. Ce type de procédé est décrit page 1289 de la référence 6.

L'étape 11 est une étape d'iodination selon la méthode de NAKATSUKA (page 1289 de la référence 6) avec de la chloramine T, du NaI*, du THF et un tampon phosphate. On obtient un dérivé 17 α-iodovinyl.

L'étape 12 est une déprotection de la fonction en position $C_3$ par hydrolyse de la fonction acétate avec de l'acétate de sodium et $Na_2CO_3$, dans du méthanol, et de l'éther(selon les détails fournis page 1289 de la référence 6). On obtient ainsi le 11 β-chlorométhyl 17 α-iodovinyl estradiol ($I^{125}$ ou $I^{123}$) [2] .

Une autre voie d'accès à l'intermédiaire [3] obtenu à l'issue de l'étape 8 est l'aromatisation de l'intermédiaire [5] , à l'issue de l'étape 8 au cours de la voie de synthèse du schéma II de l'exemple suivant. L'aromatisation de l'intermédiaire [5] intervenant après déprotection acide de son groupement éthylène dioxyde en position $C_3$. Cette aromatisation se fait par voie microbiologique avec la souche "ARTHROBACTER SIMPLEX" (ce procédé se fait selon celui décrit référence 1). Le rendement et la pureté de l'intermédiaire [5] lors de l'étape de chloration étant relativement meilleurs à ceux que l'on peut obtenir lors de la synthèse de [3] .

SCHEMA I

[1] 17α-ETHYNYL ESTRADIOL

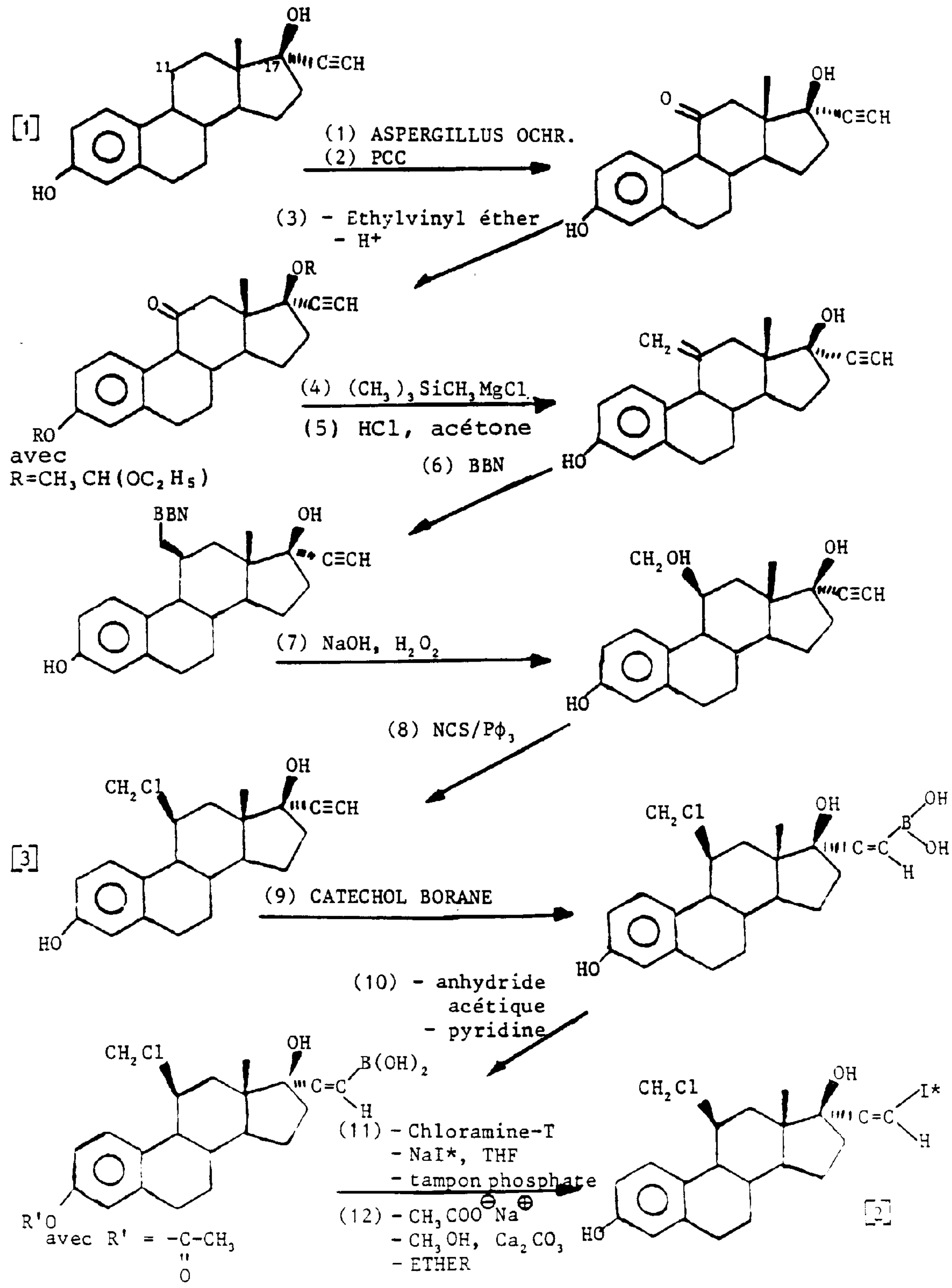


[2] = 11β-CHLOROMETHYL 17α-IODOVINYL-ESTRADIOL

**B** - Le schéma I bis ci-après reproduit une nouvelle synthèse du ligand 11 β-chlorométhyl-17 α-iodovinyl-estradiol.

L'étape 1 consiste en une préparation du $\Delta^1$ adrénostérone 17 éthylène kétal à partir du $\Delta^1$ adrénostérone (1′).

25 g $\Delta^1$ adrénostérone (84 mM) sont ajoutés sous forte agitation à un mélange de 500 ml de benzène, 25 ml d'éthylène glycol (environ 420 mM) et 1 g d'acide para-toluènesulfonique.

Le mélange est porté au reflux pendant 4 heures dans un appareil de Dean-Stark.

Le mélange réactionnel est extrait par 200 ml d'eau contenant 1 g de bicarbonate, puis par de l'eau saturée en sel, séché, puis évaporé.

On obtient une masse jaunâtre qui est lavée à chaud par 200 ml d'éther isopropylique.

Les cristaux blancs sont filtrés, puis séchés à 60°C, on obtient une masse de ± 26 g.

L'étape 2 consiste en la préparation du 11 β-hydroxy 17-éthylène kétal androsta 1,4-diène 3,17-dione (2′).

340 g de (1′) (88 mM) sont ajoutés sous azote à une suspension de 50 g (± 200 mM) dans 400 ml de tétrahydrofuranne.

Après 24 heures de réaction à température ordinaire, on ajoute 100 ml d'éther, puis 40 ml de NaOH 1N, puis 25 g de sulfate de sodium anhydre. On maintient l'agitation pendant la nuit.

Le mélange est filtré, puis le filtrat est évaporé sous vide.

La masse solide obtenue est lavée à chaud par 200 ml d'éther diisopropylique.

On obtient, après filtration et séchage, 24 g d'une poudre blanche (70,6 mM).

L'étape 3 consiste en la préparation du 3,11 ß-dihydroxy estra 1,3,5(10)-triène 17-one 17-éthylène kétal (3′).

Sous atmosphère d'azote, un mélange de 3 g de lithium enrobé dans de l'huile (environ 0,4 M), 25 g de biphényl (0,16 M) et de diphénylméthane (0,08 M) dans 360 ml de tétrahydrofuranne est porté à reflux pendant 1 heure.

Le mélange réactionnel bleu foncé, auquel on ajoute 20,7 g de (2′), est à nouveau porté à reflux pendant 30 minutes.

Après refroidissement, on ajoute 8 ml de méthane, puis 100 ml d'eau et on évapore le solvant sous vide.

Après redissolution dans la masse restante, dans l'éther, on extrait le produit formé par 300 ml de KOH 5 % qui est réacidifié avec l'acide acétique (12 ml).

Le précipité jaune est réextrait par l'acétate d'éthyle. Après évaporation du solvant, on redissout le produit à chaud dans 15 ml d'un mélange acétone et éther diisopropylique.

Après deux cristallisations, on obtient 10,5 g de produit.

Le point de fusion corrigé est : 191,1°C.

L'étape 4 consiste en la préparation du 3-benzyloxy, 11 β-hydroxy estra 1,3,5(10)-triène 17-one 17-éthylène kétal (4′).

Le mélange de 10,3 g de (3′) (30 mM), 5,2 g de $K_2CO_3$ anhydre broyés et 100 ml de méthyléthyl cétone est porté à reflux sous forte agitation pendant 1 heure.

On ajoute alors 5,4 ml (45 mM) de bromure de benzyle et le reflux est maintenu pendant 48 heures.

Après extraction, on obtient 15 g d'une huile jaunâtre.

Cette huile est engagée telle quelle dans la synthèse de (5′).

L'étape 5 consiste en la préparation du 3-benzyloxy estra 1,3,5(10)-triène 11, 17-one 17-éthylène kétal (5′).

30 mM de (4′) brut (13 g) dissous dans 50 ml de chlorure de méthylène sec sont ajoutés en une fois au mélange de 13 g de pyridinium chlorochromate en suspension dans 200 ml de chlorure de méthylène sec.

Après 3 heures d'agitation à température ambiante, on ajoute 250 ml d'éther, ce qui entraîne la précipitation d'une masse noire.

Les solvants sont décantés, on lave l'insoluble avec 4 fois 50 ml du mélange v/v $CH_2Cl_2$/éther. La phase organique est percolée sur une colonne de florisa, puis évaporée.

On obtient après purification sur silice 9,6 g d'une huile jaunâtre.

L'étape 6 est la préparation du 3-benzyloxy 11-hydroxy, 11-méthyltriméthylsilane estra 1,3,5(10)-triène 17-one 17-kétal (6′).

8,6 g de (5′) dissous dans 100 ml d'éther (20 mM) sont ajoutés rapidement à 165 ml d'une solution 1 M de chlorure de méthyltriméthylsilane de magnésium.

Le mélange est porté à reflux pendant 5 heures, puis décomposé et extrait.

Le produit brut est purifié sur silice, on obtient 5,7 g d'une huile visqueuse qui cristallise.

L'étape 7 est la préparation du 3-benzyloxy 11-méthylène 1,3,5(10)-estratriène 17-one (7′).

Après addition de 0,5 ml d'HCL concentré, une solution de 5,2 g de (6′) (10 mM) dans 50 ml d'acétone est agitée pendant 2 heures à température ambiante. Le mélange réactionnel est neutralisé par $HCO_3^-$ concentré

sous vide, extrait par $CH_2Cl_2$.

Le produit brut est recristallisé dans l'acétone.

On obtient 3,4 g de cristaux jaunâtres.

Le point de fusion corrigé est : 168,1°C.

L'étape 8 consiste en la préparation du 3-benzyloxy 11-méthylène 1,3,5(10)-estratriène 17-one, 17-kétal (8').

Une solution de 3 g de (7'), 7 mM, 100 ml de benzène, 3 ml de diéthylène glycol, 150 mg d'acide paratoluènesulfonique est refluée avec "Dean Stark" pendant 4 heures.

L'extraction puis l'évaporation des solvants donnent 3,3 g d'une huile blanche qui est employée telle quelle.

L'étape 9 consiste en la préparation du 3-benzyloxy 11-hydroxyméthyl 1,3,5(10)-estratriène 17-one 17-kétal (9').

Une solution de 2,84 g de (8') (± 6 mM) dans 20 ml de THF sec est additionnée de 0,5 ml du complexe borane oxathione (± 6 mM). Après 1 heure de réaction, on ajoute 6 ml d'éthanol, puis 4 ml de NaOH 3M (± 12 mM), enfin 10 ml de $H_2O_2$ 30 % (± 12 mM). Après une nuit à température ambiante, la réaction est extraite par $CH_2Cl_2$. On obtient une huile blanche qui cristallise rapidement (poids : 2,4 g).

L'étape 10 consiste en la préparation du 3-benzyloxy 11-chlorométhyl 1,3,5(10)-estratriène 17-one (10').

A une solution de 2 g de (9') d'environ 4 mM dans 20 ml de tétrahydrofuranne est ajoutée la suspension formée dans 30 ml de THF par la réaction de 2,1 g de triphénylphosphine (8 mM) et de 1,07 g (± 8 mM) de N-chlorosuccinimide.

Le mélange devenu limpide après à peu près 1 heure est laissé à température ambiante pendant la nuit. Le THF est évaporé sous vide, le résidu brut est redissout dans 50 ml d'acétone, auquel on ajoute 0,5 ml d'HCl concentré. On laisse sous agitation pendant 2 heures. L'extraction puis la purification sur silice donnent des cristaux blancs pour 1,02 g.

S.M. (FAB) 435 (M+1).

L'étape 11 consiste en la préparation du 3-benzyloxy 11-chlorométhyl 1,3,5(10)-estratriène 17α-éthynyl 17 β-hydroxy (11').

A une solution de 0,93 g de (10') (à peu près 2 mM) dans 10 ml de tétrahydrofuranne, on rajoute 400 mg (± 4 mM) du complexe éthylènediamine - Acétylure de lithium. Après 4 heures de réaction, on réajoute 400 mg de réactif. On laisse réagir la nuit à température ambiante.

L'extraction par $CH_2Cl_2$ puis la purification sur silice nous donnent un solide légèrement jaunâtre d'environ 500 mg (1,02 mM).

L'étape 12 consiste en la préparation du 11-chlorométhyl 3,17 β-dihydroxy 17α-éthynyl 1,3,5(10)-estratriène (12').

440 mg de (11') 0,9 mM sont dissous à froid dans 20 ml de chlorure de méthylène.

On ajoute alors 4,5 ml (4,5 mM) d'une solution 1 M du complexe $BF3/(CH_3)_2S$. Le mélange réactionnel est agité à froid pendant 45 minutes.

L'extraction puis la purification sur silice nous donnent une huile qui cristallise avec une masse d'environ 150 mg (0,375 mM).

L'étape 13 consiste en la préparation du dérivé (E)-11-chlorométhyl-3,17 β-dihydroxy-17 α-(2-tributylstannylvinyl)-1,3,5(10)-estratriène (13').

A une solution de 50 mg de (12'): 0,15 mM dans 1 ml de THF, on ajoute, sous $N_2$, 150 μl de tributylétainhydrure (à peu près 0,55 mM) et 5 mg de AIBN (à peu près 0,03 mM).

Le tube est hermétiquement fermé puis agité au bain d'huile à 70°C pendant 1 heure.

La réaction est extraite par un mélange acétate d'éthyle/eau. L'huile brute est purifiée par silice ; on obtient 55 mg d'une huile blanche.

L'étape 14 consiste en la préparation du dérivé (E)-11-chlorométhyl-3,17 β-dihydroxy-17α-(2-iodovinyl)-1,3,5(10)-estratriène (14').

A une solution de 32 mg de (13') (0,05 mM) dans 3 ml de $CH_2Cl_2$, on ajoute goutte à goutte une solution d'iode 0,1 mM dans $CH_2Cl_2$ jusqu'à persistance de la coloration rosée.

Après 30 minutes, on ajoute a peu près 10 ml d'$H_2O$ avec un peu de $NaHSO_3$, on extrait par $Et_2O$.

Le produit brut est alors purifié sur silice ; on obtient 20 g de cristaux blancs pour une masse de 20 mg.

Données spectroscopiques :

RMN ($CDcl_3$, $(CD_3)_2SO$) S 1.1 (S,13$CH_3$), 3.45 (m, CHHcl), 3.62 (dd, CHHcl), 6.27 (d, CH=CHI, J=14 Hz), 6.53 (d, H(4)), 6.66 (dd, H(2)), 6.81 (d, CH=CHI, J= 14 Hz), 7.03 (d, H(1)).

## MARQUAGE A PARTIR DU DERIVE TRIBUTYLETAIN

50 μl d'une solution du dérivé (E)- 17α-tributylétainvinyl-11 β-chlorométhylestradiol (13' du schéma 1 bis)

dans l'éthanol absolu à 1 mg/ml sont ajoutés à 1 mCi de $NaI^{123}$ (10 µl dans $H_2O$ pH 7-11) contenu dans un tube d'environ 500 µl avec bouchon à visser. On ajoute 10 µl d'une solution de chloramine-T à 1 mg/ml et on agite virougeusement pendant 15 secondes. Ensuite on ajoute 10 µl d'une solution de $Na_2S_2O_5$ à 2 mg/ml et 200 µl de tampon phosphate pH 7,4. Le mélange est passé sur cartouche SPE C18 (1 ML, BAKER) et le stéroïde est élué avec 1 ml d'éthanol. Après évaporation du solvant, le stéroïde marqué est purifié sur colonne HPCL µ BONDAPAK C18 avec phase mobile 50 % $H_2O$ et 50 % d'acétonitrile. Le temps de rétention est de l'ordre de 17 minutes.

SCHEMA 1BIS

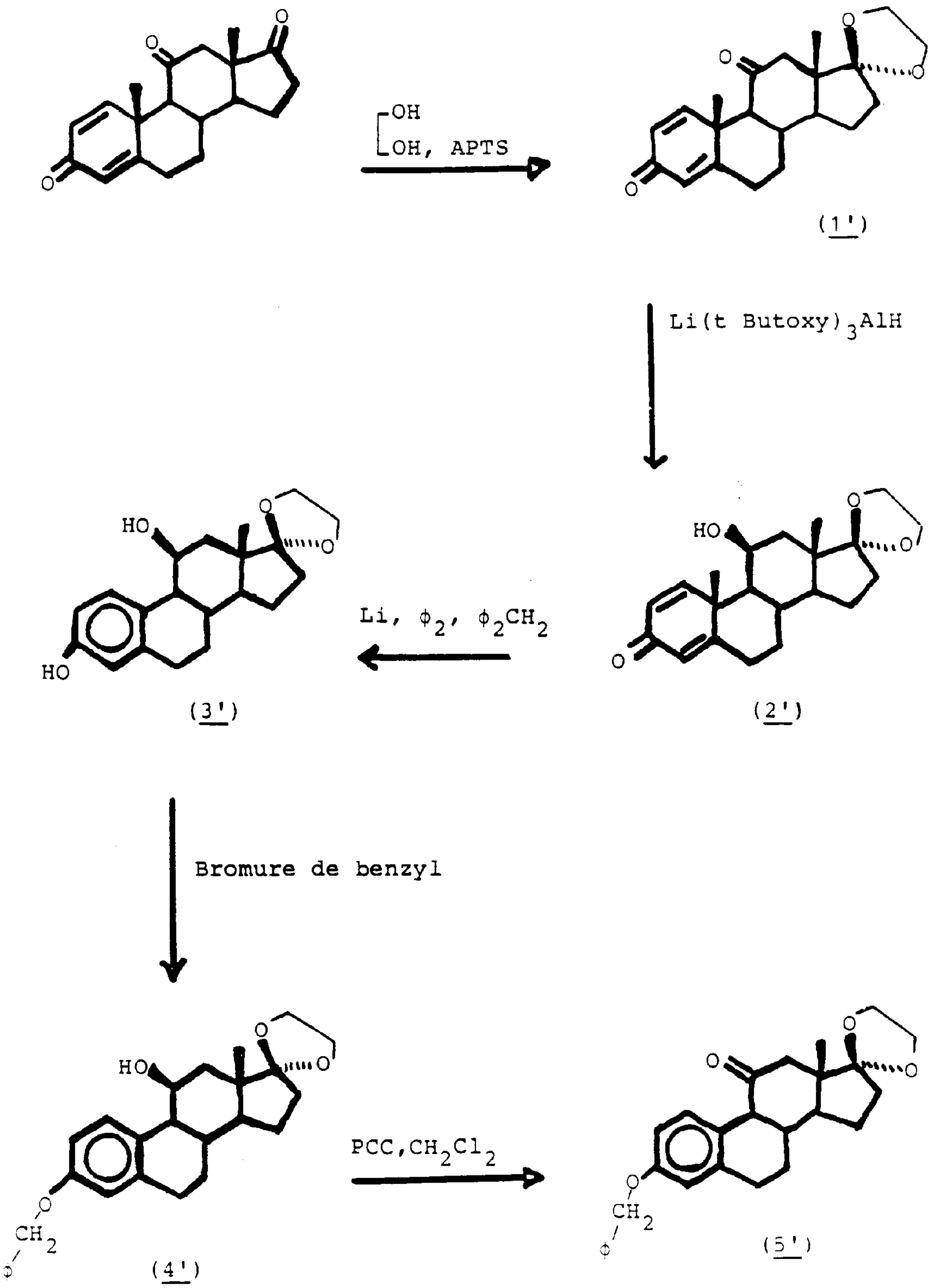


(1')

(2')

(3')

(4')

(5')

SCHEMA 1BIS suite

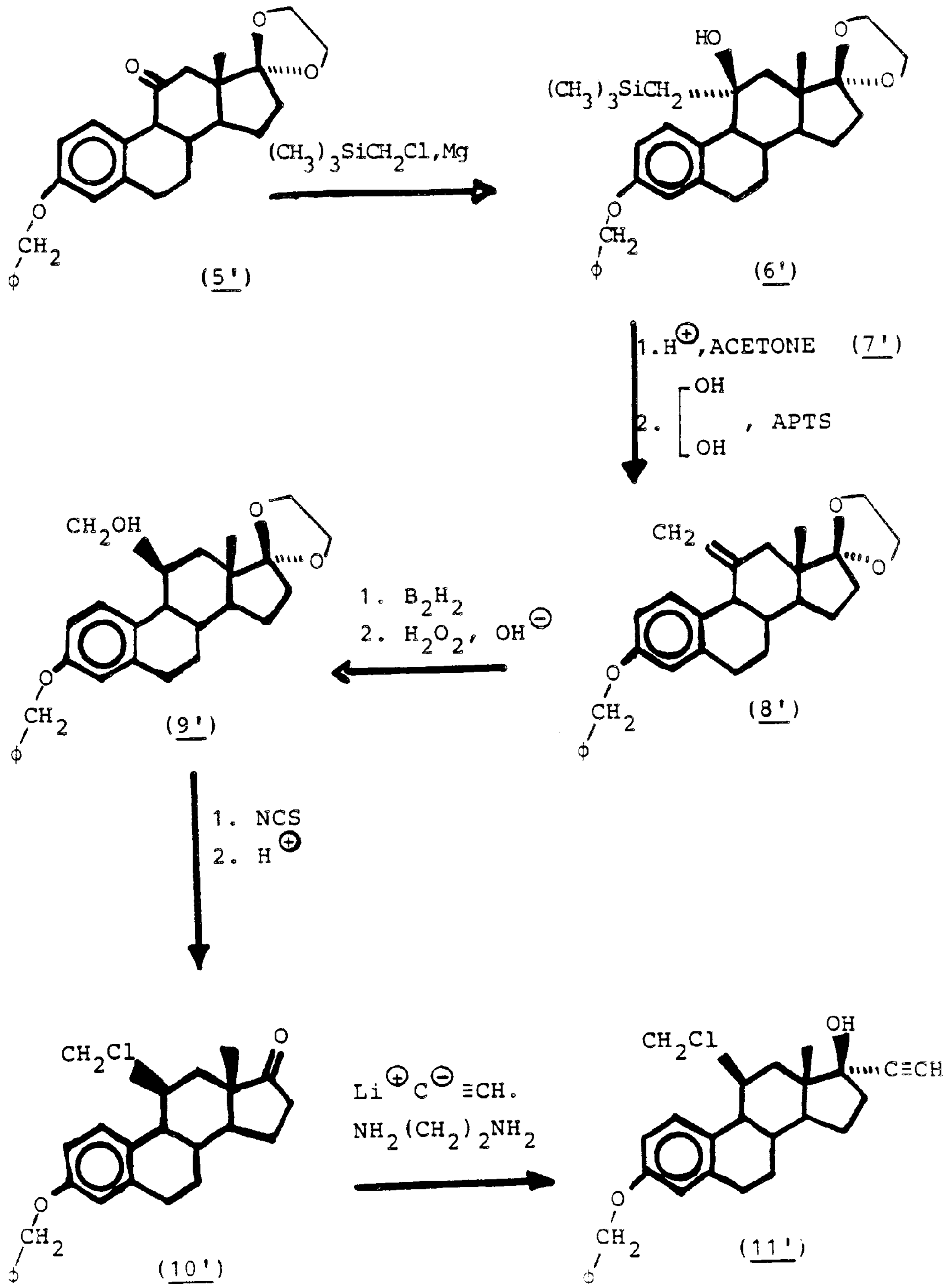


(5') (6') (8') (9') (10') (11')

SCHEMA 1BIS fin

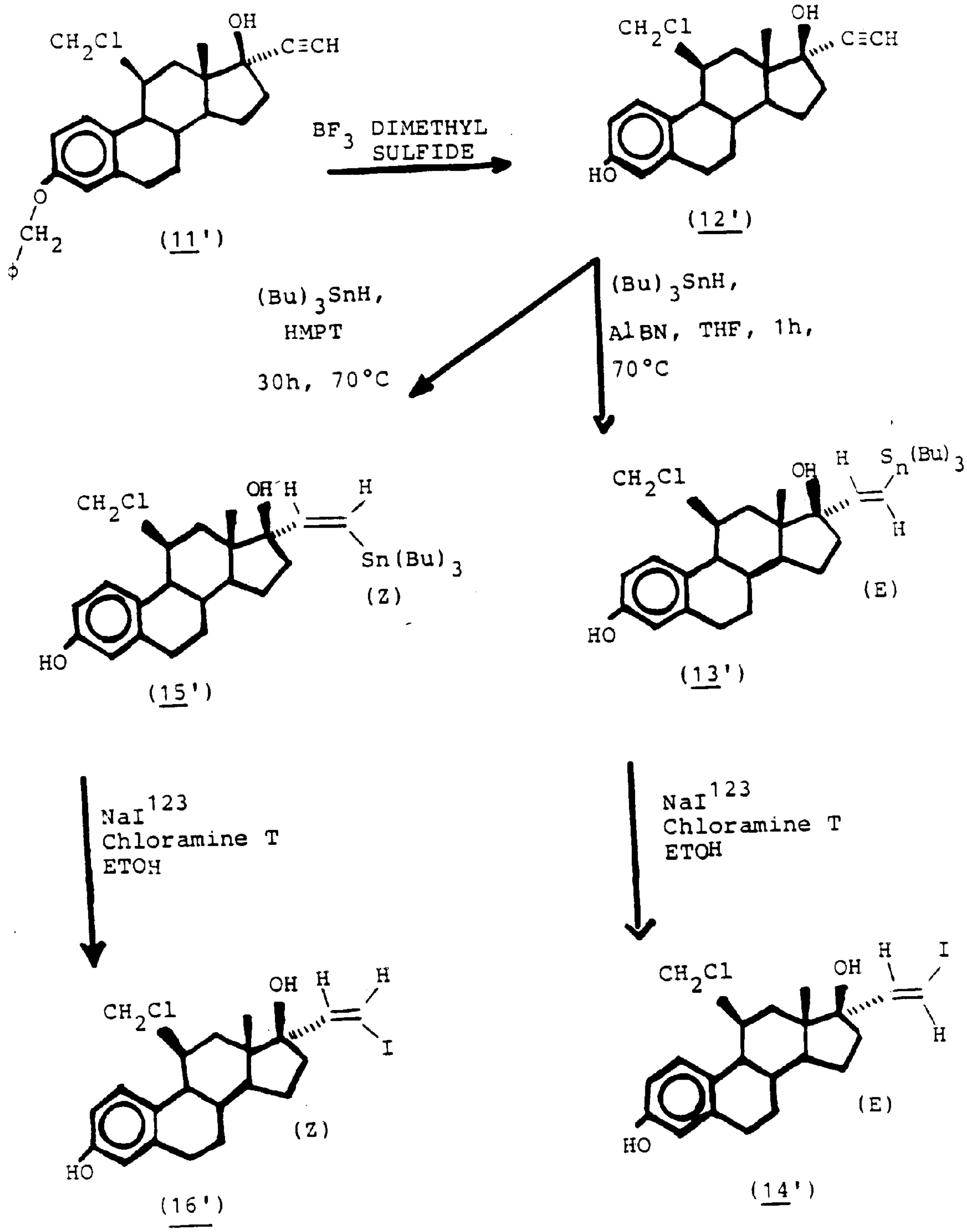


(11') (12') (15') (13') (16') (14')

## Exemple IV : LIGANDS SPECIFIQUES DU RECEPTEUR PROGESTAGENE

La voie de synthèse de dérivés 11 β-chlorométhyl 17 α-iodovinyl 17 β-hydroxy 19-nor 4-androstène 3-one est décrite en référence au schéma 11 ci-après.

Le produit de départ est le 17 α-éthynyl-17 β-hydroxy-19-nor-4-androstèn-3-one (NORETHINDRONE) [4].

Dans ce schéma I* représente les isotopes $I^{125}$ ou $I^{123}$.

L'étape 1 est une étape de protection de la fonction cétone en $_3$ par une fonction éthylène dioxy. On utilise, à cet effet, l'éthylène glycol, l'acide p-toluènesulfonique et l'orthoformate d'éthyle et $CH_2Cl_2$ comme solvant (détails de ce type de procédé sont fournis page 39 de la référence 3).

L'étape 2 est une étape de protection de la fonction hydroxyle en position 17 α protection par une fonction 1-éthoxy-éthyl-éther (selon l'étape 3 du schéma I de l'exemple I).

L'étape 3 est une étape d'hydroxylation par voie microbiologique 11 α selon le procédé de l'étape 1 du schéma I.

L'étape 4 est une étape d'oxydation avec pyridinium chlorochromate (PCC) en condition neutre par tamponnage avec l'acétate de sodium selon un type de procédé décrit(synthèse du CITRONELLAL page 2649 de la référence 7 ). On obtient ainsi une fonction cétone en position $C_{11}$.

L'étape 5 consiste en une méthylénation de la position $C_{11}$ par réaction de WITTIG utilisant le bromure de méthyltriphényl-phosphonium et le NaH dans du DMSO, conformément au procédé décrit page 39 de la référence 3.

Les étapes 6, 7 et 8 sont conformes aux étapes 6, 7 et 8 du schéma I. De même pour les étapes 9 et 10 qui sont conformes aux étapes 9 et 11 respectivement du schéma I.

L'étape 11 du présent procédé consiste en une déprotection par HCl dans l'acétone conformément au mode opératoire décrit page 334 de la référence 2 pour donner le composé [6] 11 β-chlorométhyl-17 α-iodovinyl-17 β-hydroxy-19-nor-4-androstèn-3-one.

SCHEMA II

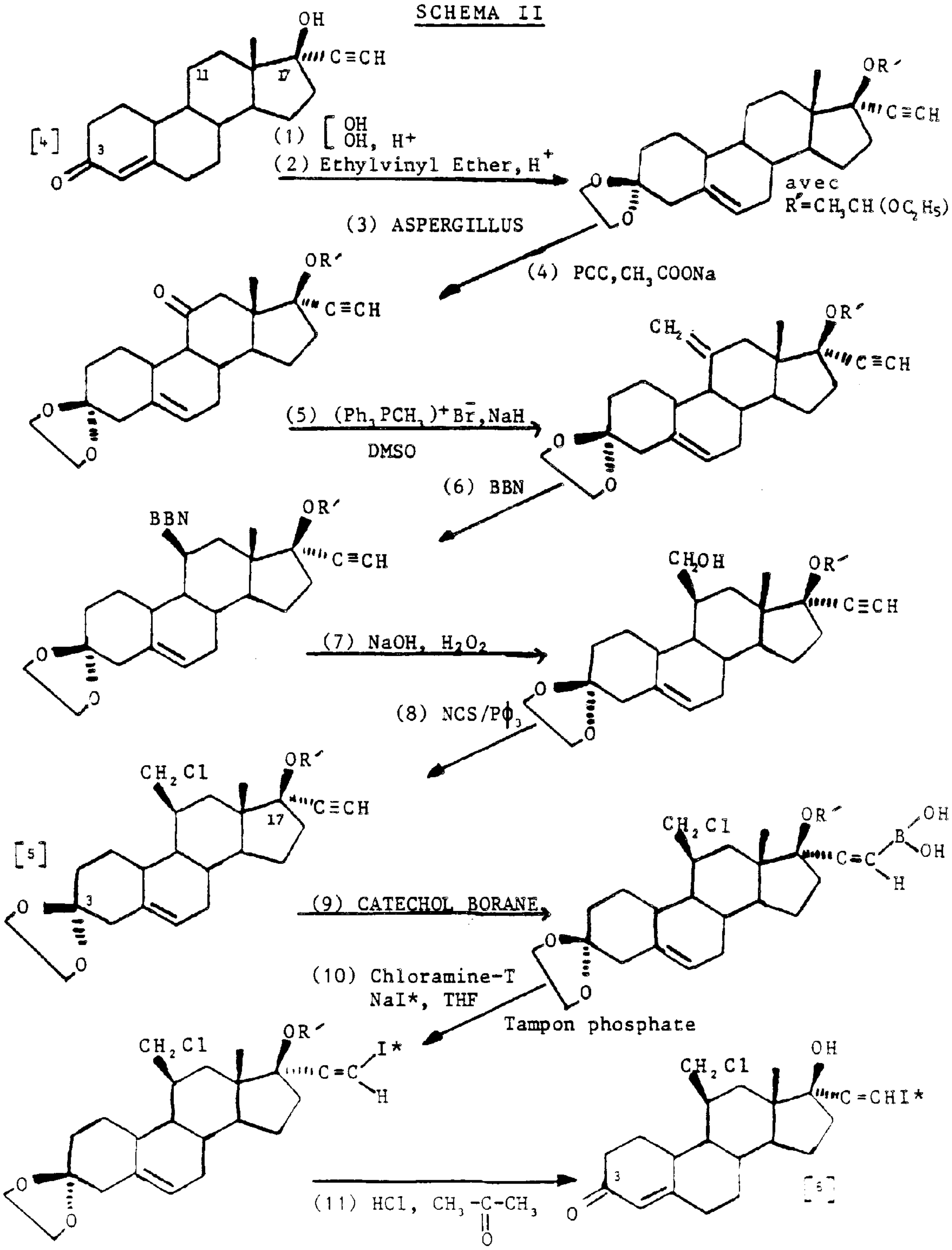


Exemple V : LIGANDS SPECIFIQUES DU RECEPTEUR ANDROGENE

La voie de synthèse d'un dérivé 11 β-chlorométhyl-17 α-méthyl-18-iodotestostérone [8] est présentée sur le schéma III ci-après.

Le produit de départ est un dérivé 11 α-hydroxy 17 α-méthyl testostérone [7].

L'étape 1 est une étape de protection conforme à celle de l'étape 1 du schéma II de l'éthylène glycol en milieu acide.

L'étape 2 est une étape d'oxydation à l'étape 4 du schéma II du pyridinium chlorochromate en condition neutre avec un tampon acétate de sodium.

L'étape 3 est une étape de protection conforme à l'étape 3 du schéma I.

L'étape 4 est une étape d'addition'd'un méthyle en position $C_{11}$ par action de $CH_3Li$ dans un mélange benzène/éther diéthylique conformément aux détails fournis page 39 de la référence 3.

L'étape 5 est une déshydratation conforme à l'étape 5 du schéma I.

L'étape 6 est une hydroboration avec du BBN comme à l'étape 6 du schéma I.

L'étape 7 est une hydroxylation comme celle de l'étape 7 du schéma I.

L'étape 8 est une chloration conforme à l'étape 8 du schéma I avec du NCS/Pϕ3. On obtient ainsi un produit [11] 11 β-chlorométhyle.

L'étape 9 est une iodination du méthyle en position 18, par action préalable du tétraacétate de plomb et de l'iode radioactif sous reflux dans du cyclohexane suivi d'addition du stéroide [11] et de l'azodisobutyronitrile selon le procédé décrit page 506 de la référence 5. On obtient ainsi la 11 β-chlorométhyl 17 α-méthyl 18-iodo-testostérone [8].

Les dérivés analogues [10] et [9] sont formés à partir du produit d'hydroxylation microbiologique, comme à l'étape 1, du dérivé 17 α-méthyl-19-nor-testostérone et de la mibolérone (7 α, 17 α-diméthyl-19-nor-testostérone) respectivement, et subissent les mêmes étapes de 4 à 9 du présent schéma.

La mibolérone et la 17 α-méthyl-19-nor-testostérone ont une meilleure affinité que la testostérone pour le récepteur androgène.

En outre, la mibolérone décrite dans le brevet US 3 341 557 a une affinité plus faible que la testostérone pour les protéines de liaison plasmatique et une rétention nucléaire nettement plus élevée confère au dérivé [9] un ciblage de haute spécificité.

SCHEMA III

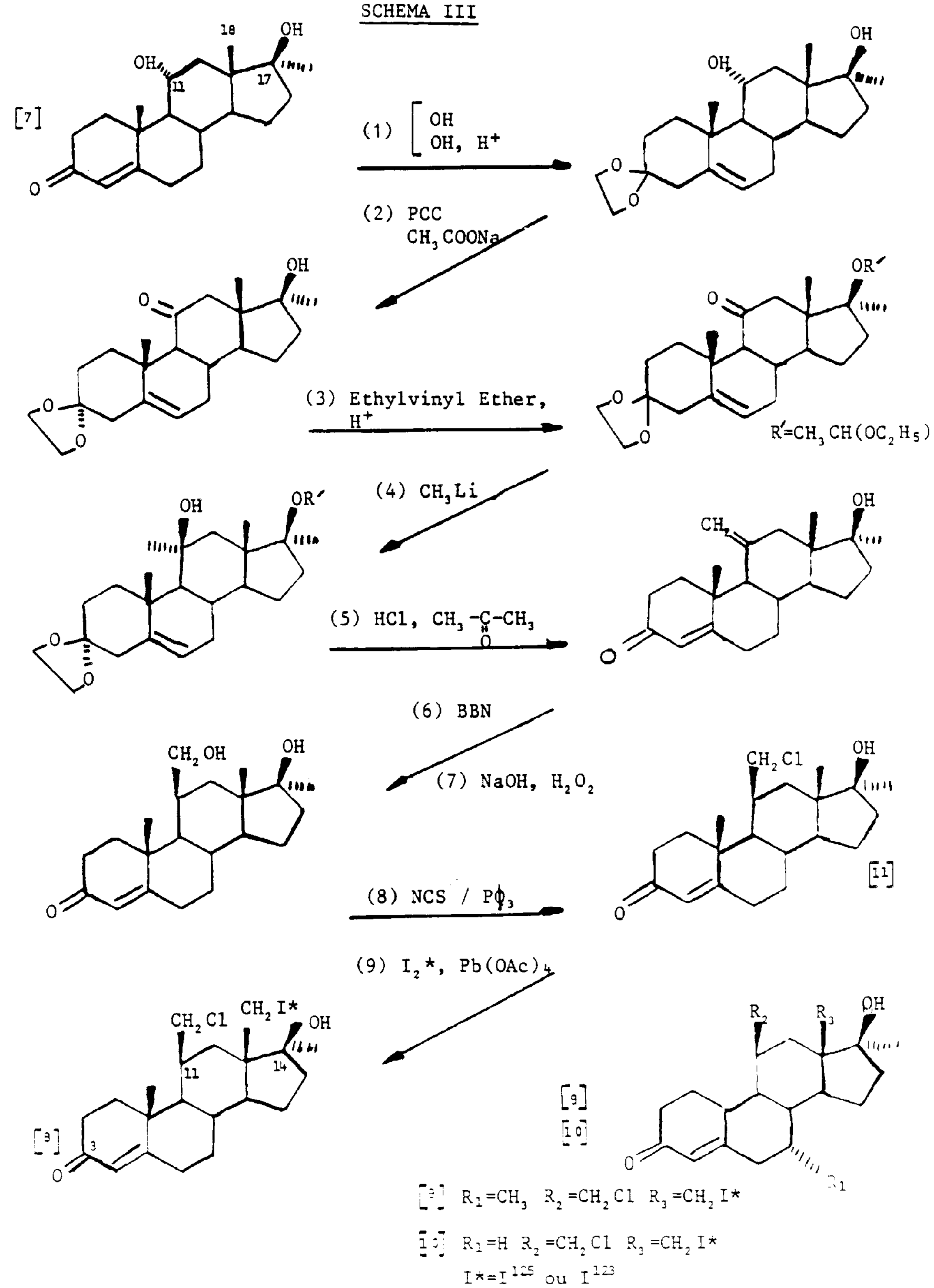
[7]
(1) OH / OH, H+
(2) PCC, CH3COONa
(3) Ethylvinyl Ether, H+
R'=CH3CH(OC2H5)
(4) CH3Li
(5) HCl, CH3-C(O)-CH3
(6) BBN
(7) NaOH, H2O2
[11]
(8) NCS / Pϕ3
(9) I2*, Pb(OAc)4
[8]
[9]
[10]
[9] R1=CH3 R2=CH2Cl R3=CH2I*
[10] R1=H R2=CH2Cl R3=CH2I*
I*=I125 ou I123

**Exemples VI** : Inhibition de la croissance de cellules malignes par le 17-bêta-iodovinyloestradiol ($I^{125}$ et $I^{123}$).

Le marquage du stéroïde a été réalisé selon le protocole décrit dans Journal of Nuclear Medicin Vol. 23 n° 5 pages 431 à 436, 1982 Robert N. HANSON et al.

La mesure de l'inhibition de la croissance de cellules malignes a été réalisée en suivant le protocole décrit par MADEDDU et al. dans ANTICANCER RESEARCH 6: 11-16(1986).

On a mesuré l'inhibition de la croissance au cinquième jour de cellules MCF7 (lignée continue de carcinome mammaire humain contenant des récepteurs pour les oestrogènes) et des cellules EP2 (lignée continue d'epithélioma pharyngien ne possédant pas de récepteurs pour les oestrogènes, inhibition par le 17-bêta-iodo-binyloerstradiol $I^{125}$ ou $I^{123}$. Il apparaît que ce ligand 17-bêta-iodovinyloestradiol marqué à l'iode 123 (une molécule d'iode pour une molécule de ligand) dans une concentration de 4,6 $10^{-10}$M correspondant à 31,3 µCi/ml donne des pourcentages de croissance pour les cellules MCF7 (5000 cellules par puits pour le contrôle) de 56,2 %, soit une inhibition de croissance de 43,8 %, et pour les cellules EP2 (contrôle 5000 cellules par puits) une croissance de 97 %, soit une inhibition de 3 % seulement.

Le même ligand comportant un iode 125 dans la même concentration de 4,6 $10^{-10}$M correspondant de 1 µCi/ml de donnait pour les cellules MCF7 (contrôle 5000 cellules par puits) une inhibition que de 10,4 % et pour les cellules EP2, le pourcentage de croissance était de 105,1 %.

En conséquence, le composé marqué à l'iode 123 présente une inhibition de la croissance très intéressante, vis-à-vis des cellules MCF7.

REFERENCES

(1) A.J. VAN DEN BROEK et al., PHARMACEUTISCH WEEKBLAD SC. ED., p. 182-183, Vol. 5, 1983,
(2) K.H. SCHÖNEMANN et al., EUR. J. MED. CHEM. CHIMICA THERAP. - p. 333-335, 15n° 4, 1980
(3) A.J VAN DEN BROEK et al., RECUEIL JOURNAL OF THE ROYAL NETHERLANDS CHEMICAL SOC. - p. 35-39, 94/2, 1975,
(4) J. DE FLINES et al., RECUEIL J. ROYAL NETH. CHEM. SOC. p. 129)138, 82, 1963,
(5) A.J. VAN DEN BROEK et al., STEROIDS - p. 481-510, 30, 1977,
(6) I. NAKATSUKA et al., J. OF MEDICINAL CHEMISTRY, p. 1287-1291, 27, n° 10, 1984,
(7) E.J. COREY et W. SUGGS, TETRAHEDRON LETTERS, p. 2647-2650, n° 31, 1975,
(8) C.A. BROWN, R.A. COLLMAN, J. ORG. CH., P 2328, 44, 1979.

**Revendications**

1. Ligands spécifiques de récepteurs d'hormones stéroïdes utiles pour la thérapie ciblée ou l'imagerie notamment du cancer présentant un squelette structurel de base ayant pour formule

(I)

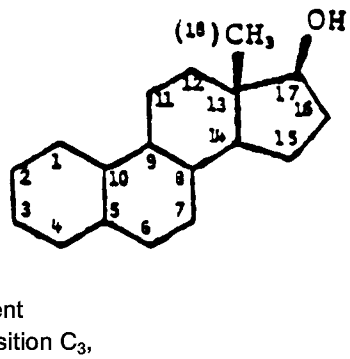

caractérisés en ce qu'ils comportent notamment

a une fonction hydroxyle ou cétone en position $C_3$,

b une fonction chlorométhyle en β sur la position $C_{11}$,

c une fonction vinyle ou méthyle en α sur la position $C_{17}$ et,

d un iode radioactif substitué sur un groupement alkyl ou alkényl rattaché au squelette, notamment un méthyl ou un vinyl.

2. Ligands utiles pour la thérapie ciblée, notamment du cancer selon la revendication 1, caractérisés en ce que l'iode radioactif est l'isotope $I^{125}$ ou $I^{123}$.

3. Ligands selon la revendication 1 ,utiles pour l'imagerie médicale, notamment du cancer, caractérisés en ce que l'iode radioactif est l'isotope $I^{123}$ .

4. Ligands selon l'une des revendications 1 à 3, caractérisés en ce que l'iode radioactif est situé sur une double liaison d'un vinyl en α sur la position $C_{17}$.

5. Ligands selon l'une des revendications 1 à 3, caractérisés en ce que l'iode radioactif est situé sur un méthyle angulaire positionné en $C_{18}$.

6. Ligands selon la revendication 4, caractérisés en ce que la fonction en position $C_3$ est un groupe hydroxyle attaché à un cycle aromatique.

7. Ligands selon la revendication 4, caractérisés en ce que la fonction en position $C_3$ est une fonction cétone conjuguée à une double liaison en $C_4$-$C_5$.

8. Ligands selon la revendication 5, caractérisés en ce que la fonction en position $C_3$ est une fonction cétone conjuguée à une double liaison en $C_4$-$C_5$.

9. Ligands selon la revendication 8, caractérisés en ce qu'il comporte en outre un méthyle en $C_7$.

10. Ligands selon la revendication 6, caractérisés en ce que c'est un 11 β-chlorométhyl 17 α-iodovinyl-estradiol de formule

(II)

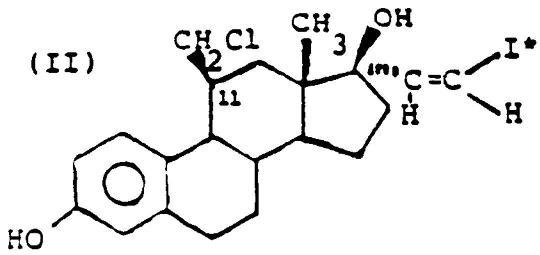

11. Ligands selon la revendication 7, caractérisés en ce que c'est un 11 β-chlorométhyl 17 α-iodovinyl 17 β-hydroxy-19-nor-4-androstèn-3-one de formule

(III)

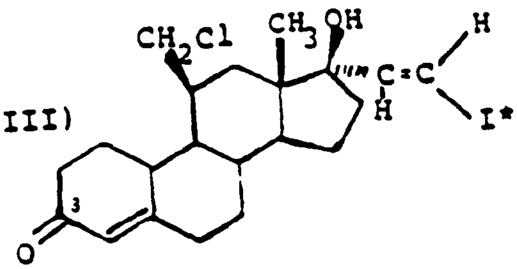

12. Ligands selon la revendication 8, caractérisés en ce que c'est un 11 β-chlorométhyle 17 α-méthyle 18-iodotestostérone ou une préférence 19-nor testostérone de formule

(IV)

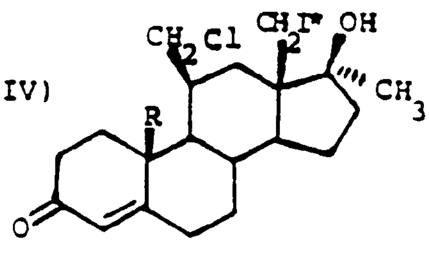

avec R = $CH_3$ ou de préférence H.

13. Ligands selon la revendication 8, caractérisés en ce que le 11 β-chlorométhyl-17 α-méthyle 18-iodo 7 α-méthyl 19-testostérone ou, de préférence, 19-nor testosterone a pour formule

(V)

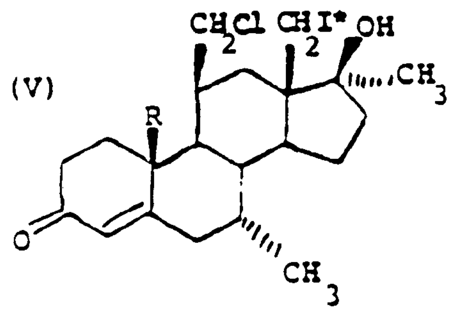

avec R = $CH_3$ ou de préférence H.

## Claims

1. Ligands specific for steroid hormone receptors which are useful for the targeted therapy or the imaging, in particular of cancer, possessing a parent structural skeleton of the formula:

(I)

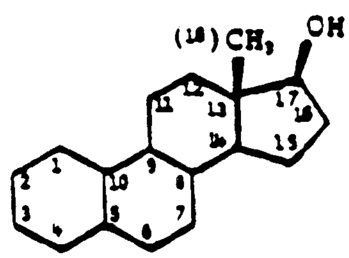

which ligands contain, in particular:

a a hydroxyl or keto group at the $C_3$-position,

b a β-oriented chloromethyl group on the $C_{11}$-position,

c an α-oriented vinyl or methyl group on the $C_{17}$-position, and

d a radioactive iodine substituted on an alkyl or alkenyl group attached to the skeleton, in particular a methyl or a vinyl.

2. The ligands which are useful for the targeted therapy, in particular, of cancer as claimed in claim 1, in which the radioactive iodine is the isotope $I^{125}$ or $I^{123}$.

3. The ligands as claimed in claim 1, which are useful for the medical imaging, in particular, of cancer, in which the radioactive iodine is the isotope $I^{123}$.

4. The ligands as claimed in one of claims 1 to 3 in which the radioactive iodine is situated on a double bond of an α-oriented vinyl on the $C_{17}$-position.

5. The ligands as claimed in one of claims 1 to 12, in which the radioactive iodine is situated on an angular methyl positioned at $C_{18}$.

6. The ligands as claimed in claim 4, in which the group at the $C_3$-position is a hydroxyl group attached to an aromatic ring.

7. The ligands as claimed in claim 4, in which the group at the $C_3$-position is a keto group conjugated with a $C_4$-$C_5$ double bond.

8. The ligands as claimed in claim 5, in which the group at the $C_3$-position is a keto group conjugated with a $C_4$-$C_5$ double bond.

9. The ligands as claimed in claim 8, which contains (sic), in addition, a methyl at $C_7$.

10. The ligands as claimed in claim 6, which is (sic) an 11β-chloromethyl-17α-iodovinylestradiol of formula

(II)

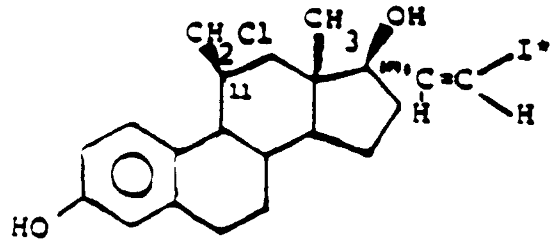

11. The ligands as claimed in claim 7, which is (sic) an 11β-chloromethyl-17α-iodovinyl-17β-hydroxy-19-nor-4-androsten-3-one of formula

(III)

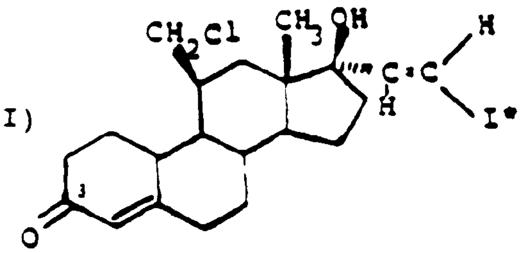

12. The ligands as claimed in claim 8, which is (sic) an 11β-chloromethyl-17α-methyl-18-iodoteatosterone or preferably -19-nortestosterone of formula

(IV)

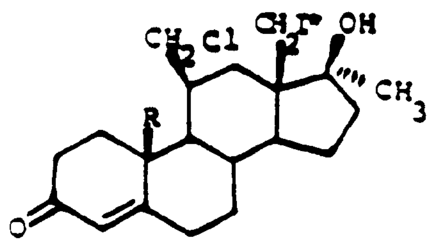

with R = $CH_3$ or preferably H.

13. The ligand as claimed in claim 8, in which 11β-chloromethyl-17α-methyl-18-iodo-7α-methyl-19-testoaterone (sic) or preferably -19-nortestostorono is of the formula

(V)

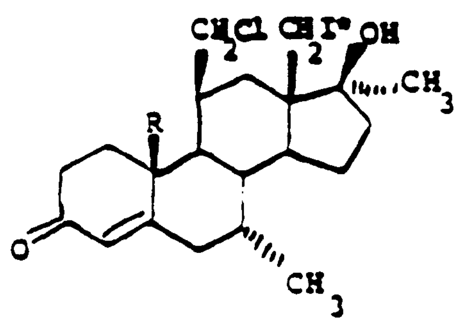

with R = $CH_3$ or preferably H.

## Patentansprüche

1. Liganden, spezifisch für Rezeptoren für Steroidhormone, geeignet zur Target-Therapie oder Visualisierung insbesondere von Krebs, mit einem Basis-Strukturgerüst der Formel

(I)

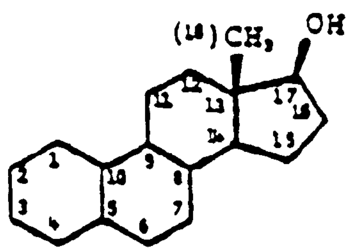

dadurch gekennzeichnet, daß sie insbesondere umfassen

a eine Hydroxyl- oder Ketonfunktion in $C_3$-Position

b eine Chlormethylenfunktion in β-Stellung in der $C_{11}$-Position

c eine Vinyl- oder Methylfunktion in α-Stellung in der $C_{17}$-Position und

d ein radioaktives Jod, substituiert an einer an das Gerüst gebundenen Alkyl- oder Alkenyl-, insbesondere einer Methyl- oder Vinyl-Gruppe.

2. Liganden, geeignet zur Target-Therapie, insbesondere von Krebs, nach Anspruch 1, dadurch gekennzeichnet, daß das radioaktive Jod das Isotop $I^{125}$ oder $I^{123}$ ist.

3. Liganden nach Anspruch 1, geeignet zur medizinischen Visualisierung, insbesondere von Krebs, dadurch gekennzeichnet, daß das radioaktive Jod das Isotop $I^{123}$ ist.

4. Liganden nach einem der Ansprüche 1 bis 3, dadurch gekennzeichnet, daß das radioaktive Jod an einer Doppelbindung eines Vinyls in α-Stellung in der $C_{17}$-Position vorhanden ist.

5. Liganden nach einem der Ansprüche 1 bis 3, dadurch gekennzeichnet, daß das radioaktive Jod an einem angularen Methyl in $C_{18}$-Position vorhanden ist.

6. Liganden nach Anspruch 4, dadurch gekennzeichnet, daß die Funktion in $C_3$-Position eine Hydroxylgruppe, gebunden an einen aromatischen Ring, ist.

7. Liganden nach Anspruch 4, dadurch gekennzeichnet, daß die Funktion in $C_3$-Position eine Keton-Funktion, konjugiert mit einer Doppelbindung in $C_4$-$C_5$ ist.

8. Liganden nach Anspruch 5, dadurch gekennzeichnet, daß die Funktion in $C_3$-Position eine Keton-Funktion, konjugiert mit einer Doppelbindung in $C_4$-$C_5$ ist.

9. Liganden nach Anspruch 8, dadurch gekennzeichnet, daß sie außerdem ein Methyl in $C_7$ aufweisen.

10. Liganden nach Anspruch 6, dadurch gekennzeichnet, daß es sich um ein 11β-Chlormethyl-17α-jodovinyl-östradiol handelt mit der Formel

(II)

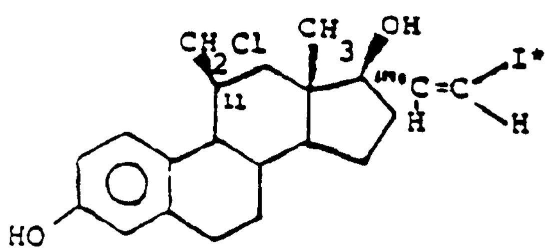

11. Liganden nach Anspruch 7, dadurch gekennzeichnet, daß es sich um ein 11β-Chlormethyl-17α-jodovinyl-17β-hydroxy-19-nor-4-androsten-3-on handelt mit der Formel

(III)

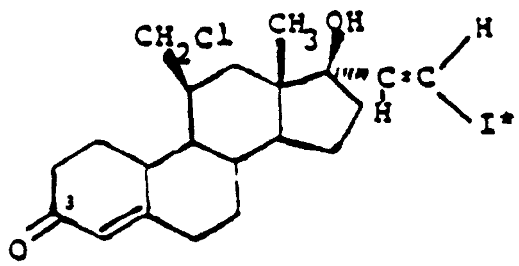

12. Liganden nach Anspruch 8, dadurch gekennzeichnet, daß es sich um ein 11β-Chlormethyl-17α-methyl-18-jodotestosteron oder vorzugsweise 19-Nor-testosteron der Formel

(IV)

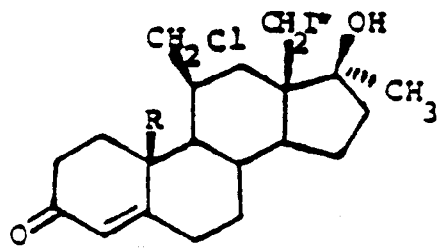

handelt, worin R = $CH_3$ oder vorzugsweise H.

13. Liganden nach Anspruch 8, dadurch gekennzeichnet, daß das 11β-Chlormethyl-17α-methyl-18-jodo-7α-methyl-19-testosteron oder vorzugsweise 19-Nor-testosteron die Formel

(V)

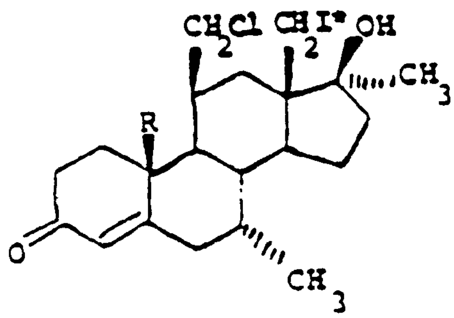

hat, worin R = $CH_3$ oder vorzugsweise H.